# EUROPEAN PATENT APPLICATION

(11) **EP 0 689 050 A1**
(43) Date of publication of application: **27.12.1995**
(21) Application number: 94201757.5
(22) Date of filing: 20.06.1994
(51) Int. Cl.: G01N 27/416

(54) **Packaged reference solution**

(71) Applicant: EURO-TROL B.V., NL-6702 EA Wageningen (NL)
(72) Inventor: Maas, Bart H.A., NL-3561 HL Utrecht (NL); Maas, Anton H.J., NL-3571 ZB Utrecht (NL); Sprokholt, Ron, NL-6666 XL Heteren (NL)
(74) Representative: Kroesen, Johan Albert Carel

(57) **Abstract**

Samples to be analysed and califration solutions for such analyses have a limited keepablity; they lose carbondioxyde and/or oxygen or take them up from the surrounding air. Analysing for several physiologically important parameters becomes unreliable. The invention provides a solution for this problem: glas syringes and cartridges capped with gas-impervious polymer, e.g. rubber. If desired they may be wrapped in an additional foil.

## Description

This invention relates to a packaged solution for blood-gas analysis comprising an aqueous solution having known concentrations of solutes and gases but no gas phase contained in a sealed, impermeable container.

Such a packaged solution is known from US 4,960,708 according to which it is contained in a sealed, flexible and impermeable container; the total gas pressure should be 550-800 mmHg at 37°C. Furthermore this solution contains a highly diffusible gas, such as hydrogen or helium (helium being preferred because of its uninflammability). When the package is opened microbubbles will not appear, which could drastically alter the composition of the gas phase.

In a hermetically closed container, such as a sealed ampoule, liquid and gas (e.g. an aqueous solution and oxygen and/or carbon dioxide) are not at equilibrium. That is the general experience. This necessitates agitating the container profoundly before it is opened to extract its contents. Without that precaution the solution will not contain the expected amount of gas, the usual deviation being up to 4-6 mmHg, (on an actual tension of 40-60 mmHg). It is obvious that such a deviation from the expected contents will cause a lot of nuisance and errors, and is absolutely unacceptable.

This possible source of errors would become extinct if the analysis solution could be kept in absence of gas phase. And that would also terminate the temperature dependence of the gas concentration of said solution, while the solubility coefficient of the gas decreases with increasing temperature.

The no-return plastic syringe regrettably is not gas impervious. It has been observed that its liquid content will not only take up oxygen through the latitude between plunger and cylinder, but even also through the cylinder wall.

The container according to US 4,960,708 is either a plastic or laminate bag or a metal tube. It is obvious that producing a plastic bag that is really gas-impervious is very hard to realize, if not impossible. Additionally, if the inner pressure is over the ambient pressure opening the container will inescapably be accompanied by spillage and the like accidents. It is significant that nothing is said about that in the only example of US 4,960,708.

Now it has been found that a packaged reference solution for blood gas analysis having the desired characteristics is realized, said container is a glass cylinder open at one end and accomodating a plunger with stem and having at an opposite end a lesser opening, both the plunger and said lesser opening being capped with gas-impervious polymer. Hereafter this container is referred to as "syringe". Alternatively such a container is a glass cylinder open at one end and accomodating a plunger and having at an opposite end an opening with the same diameter capped with a stopper, both plunger and stopper made of a gas-impervious polymer, hereafter referred to as "cartridge". Both syringe or cartridge might be packed in a gas-impervious foil to obtain a shelf-life of many years.

There is no need of helium, hydrogen or any other gas, of whatever molecular weight. It will not be necessary to maintain any pressure in or on the container or the surrounding thereof. But the space between syringe or cartridge and foil, if present, may be filled with any gas or gas mixture, and there may also be a vacuum in it. The syringe or cartridge is simple to produce and simple in use: The optional rubber foil and closure of the lesser opening are removed and by activating the plunger the contents of the cylinder is transferred to an appropriate destination, e.g. a blood gas analyzer. The cartridge can be used by removing the foil and use it in a pistol (a holder which has on one side a needle to penetrate the stopper and on the other side a stem). The combination of cartridge and pistol has the same function as the syringe with the exception that the cartridge is a disposable and the pistol can be used for many analyses.

Filling the syringe or cartridge may be done from any of the two sides, either by aspirating the solution through the lesser opening (the plunger being drawn backwards) or by pouring the solution in the open cylinder (the lesser opening being covered by the polymer closure). Storage will be simple and may occur at any temperature, e.g. as well at 50°C as at 0°C; as there is no gas phase there will be no worry about the equilibrium between the solution and the gas phase. The solution, being in a closed and air-tight container, will not lose an unknown amount of gas, so that the amount of gas and the gas pressures in said solution will reliably be at their expected values.

Additionally there will be neither difficulty in opening the syringe or cartridge nor risk of broken glass, generated by opening a sealed ampoule.

A syringe or cartridge for the packaged reference solution according to the invention is sketched in the accompanying drawing. The size may vary as desired, it may contain as little as a few drops and as much as 100 cm³ or even more; it is made of glass, which may be any kind of available glass. Apart from the polymer capping of the plunger and the polymer capping of the lesser opening of the syringe or the stopper of the cartridge it is made of glass, which may be any kind of available glass. Its thickness will be such as to allow normal handling.

The polymer of the major and the lesser cap of the syringe and the plunger and stopper of the cartridge must be impervious to gas, e.g. its gas penetration constant α of said gas is expressed in litres of gas per second at a pressure difference of 1 atmosphere (101.325 pascals) could be less than 3.10⁻¹²H, wherein H is the Henry constant for the solubility of the gas (oxygen or carbon dioxide) in the solution in litre per litre (at any pressure). With such a penetrability a fleece of 1 square cm having a thickness of 1 mm will allow in 1 year of time at a pressure difference of 0.1 atmosphere (10.132 pascals) to pass 0.01 cm³ of gas, to change the gas concentration of 1cm³ of solution by 1%. The Henry constante of oxygen and carbon dioxide at 20°C are 0.031 and 0.87 respectively.

In general the polymer will be rubber. A very suitable kind of rubber that meets the above demand is the so-called bromobutyl or chlorobutyl rubber. It is e.g. available from PharmaGummi. It allows normal handling and normal sterilization. It does meet the above-mentioned criterion of a low gas permeability coefficient, but other kinds of polymers showing a sufficiently low permeability are also suitable.

The cap of the plunger should be a piece of the same dimensions as the inner cross-section of the cylinder, and its thickness may be anything above about 1 mm. e.g. it is 5 mm thick.

The above container will be primarily used for storing a reference solution. However, it may also be used to keep and transfer the solution to be analysed. Another option is storing a series of solutions for the verification of the linearity (of the analysis apparatus).

### EXAMPLE 1

A syringe was made in an usual way from customary, commercially available glass, its sizes were as depicted in figure 1. The inner diameter of the cylinder was 8.0 mm, its length was 5.0 cm. The reference solution was meant for blood gas analysis, was buffered at a pH of 6.8 and it contained electrolytes, glucose and lactate, as dissolved gas it contained CO₂ to a tension of 150 mmHg and O₂ to a tension of 30 mmHg, the balance being nitrogen.

Rubber caps and plunger caps were made from bromobutyl rubber obtained from Pharma-Gummi.

### EXAMPLE 2

A cartridge was made in a usual way from customary, commercially available glass, its sizes being as indicated in figure 2. The reference solution was buffered at a pH of 7.8 and it contained electrolytes, glucose. To this solution protein was added, to wit a solution of hemoglobin derivatives. As dissolved gas it contained CO₂ to a tension of 20 mmHg and O₂ to a tension of 400 mmHg, the balance being nitrogen.

The caps and cylinder were made from chlorobutyl rubber obtained from Pharma-Gummi.

## Claims

1. Packaged reference solution for blood-gas analysis, comprising an aqueous solution having known concentrations of solutes and gases but no gas phase, contained in a sealed, impermeable container, characterized in that the container is a glass cylinder open at one end and accomodating a plunger with stem and having at an opposite end a lesser opening, and in that both the plunger and the lesser opening are capped with gas-impervious polymer.

2. Packaged reference solution for blood-gas-analysis, comprising an aqueous solution having known concentrations of solutes and gases but no gas phase, contained in a sealed, impermeable container, characterized in that the container is a glass cylinder open at both ends, one of which is capped with a transposable plug and the other with a cap, both plug and cap being made of gas-impervious polymer.

3. Packaged reference solution according to claim 1 or 2, the polymer of stopper and caps being chlorobutyl or bromobutyl rubber.

4. Packaged reference solution according to claim 1, 2 or 3, the contents of which is a calibration solution or a series of solutions for the verification of the linearity.

5. Packaged reference solution according to claim 1, 2 or 3, the contents of which is a reference solution for blood gas analysis containing electrolytes, glucose and lactate.

6. Packaged reference solution according to claim 1, 2 or 3, in which protein was added to the solution.

7. Packaged reference solution according to any of the preceding claims, in which any type of container is sealed in a gas-impervious foil to attain a shelf-life of many years.
